# EUROPEAN PATENT SPECIFICATION

(11) **EP 0 410 986 B1**
(45) Date of publication and mention of the grant of the patent: **20.10.1993**
(21) Application number: 89904140.4
(22) Date of filing: 13.03.1989
(51) Int. Cl.: A61F 7/00

(54) **SCALP COOLING APPARATUS**
VORRICHTUNG ZUR KÜHLUNG DER KOPFHAUT
APPAREIL DE REFROIDISSEMENT DU CUIR CHEVELU

(30) Priority: 16.04.1988 GB 8809029
(43) Date of publication of application: 06.02.1991
(73) Proprietor: GREATER GLASCOW HEALTH BOARD, Glasgow G4 9LF (GB)
(72) Inventor: MAXTED, Kenneth, John, Glasgow G77 5NF (GB); MOUNTFORD, Neville, Lincoln LN6 0QW (GB)
(74) Representative: Naismith, Robert Stewart
(86) International application number: GB8900250
(87) International publication number: WO8909583

(56) References cited:
- EP-A- 0 076 080
- WO-A-82/04184
- DE-A- 2 247 406
- FR-A- 2 195 421
- US-A- 2 817 340
- US-A- 3 307 553
- US-A- 3 587 577
- US-A- 3 866 612
- US-A- 4 572 188

## Description

The present invention relates to a cooling apparatus and especially, but not exclusively, to scalp cooling apparatus and to a method of cooling a scalp which is particularly suitable for treating patients undergoing chemotherapy.

The use of cytotoxic agents in, for example, chemotherapy of cancer patients almost inevitably causes hair loss known as alopecia. Alopecia can be prevented by cooling the scalp because drug uptake by hair follicles is reduced as a consequence of cutaneous vascoconstriction and the inhibition of cellular metabolic pathways. Cooling can be achieved in a number of ways: by circulating chilled liquid in close thermal contact with the scalp; by the application of crushed ice packs or shaped cryogel packs; by the activation of endothermic chemical reactions, and by the circulation of cold air over the scalp.

Cold air scalp cooling has several advantages over the other cooling methods. Firstly it requires no additional thermal contact medium thus, unlike all the other methods, hair wetting is not required. The technique can be applied with very little weight being imposed on the patients head and is therefore much less tiring and uncomfortable, and thermal transfer can be optimised over the entire scalp by design of the applicator helmet.

Clinical trials have been performed using a vortex tube air refrigerator as described in the paper entitled "Adriamycin Alopecia Prevented by Cold Air Scalp Cooling" published in American Journal of Clinical Oncology (CCT) 9(5):454-457,1986 by Symonds McCormick and Maxted. This disclosure established the requirements for temperature and air flow and demonstrated that clinical results, equalling or exceeding any previously published, could be achieved without skill of application or degree of preparation required for any of the other methods.

However the use of standard refrigeration components to provide a source of cold air has a number of problems. Namely freezing of entrained moisture on the evaporator causes low efficiency due to latent heat loses and airflow obstruction and the circulating fan causes significant heat gain. The helmet and tubing design described in the above paper did not permit sufficient free flow of air to avoid significant pressure drops.

US-A 4 572 188 discloses a scalp airflow cover in the form of an inflatable rounded cap comprising the features set out in the first part of claim 1, which cap is connected to a supply of pressurized air which may be heated or cooled. The air inflates a tube to create a seal with the head, a plurality of exit ports being provided on the inner face of the tube and from which air passes over the head and out of an exit port. The requirement for the provision of pressurized air sufficient to inflate the cap would require a relatively powerful air supply. US-A 3 587 577 discloses a header device for applying jets of fluid to the scalp of a patient. The used fluid drains through a strainer to be recycled through a heat exchanger. Collection of the fluid requires the patient to lie flat with their head over the strainer, and the patient will have to dry their hair after the operation is complete. US-A 2 817 340 discloses air-hibernating apparatus in which a hibernating bag is supplied with cooled air from a refrigerating unit which including a cooling coil and receptacle for the chloride of calcium for drying recycled air. The air drying chemicals would require to be replaced at regular intervals.

The objects of the present invention include the provision of a helmet and an improved cooling system which obviate or mitigate at least one of the aforesaid disadvantages.

In a preferred arrangement an applicator helmet has an air delivery gallery which directs air onto the scalp and hairline through a number of profile drillings of such size and profile as to minimize frictional losses in the air stream. Air is extracted at the crown of the helmet where the convergence and increasing air velocity compensates for increased air temperature so that at this point, scalp cooling is optimised by using the wind-chill effect. The interior of the helmet includes a number of spacers which are held in gentle contact with the scalp by applying a slight force in the helmet using a counterbalanced arm. This permits the helmet to remain in contact with the scalp even as the patients relaxes and slumps in the chair during treatment. The helmet is also provided with an elasticated fabric skirt to reduce air loss from the closed air recirculation circuit.

According to one aspect of the present invention there is provided a helmet for use with a recirculating scalp cooling system, said helmet comprising air inlet means and air outlet means, said air inlet means being provided by a plurality of apertures disposed around the periphery of the rim of the helmet and said air outlet means being disposed at the crown of said helmet, characterised in that spacing means are located inside the helmet for setting the distance between the helmet and the scalp and means for minimizing air loss from and to prevent entrainment of room air into the helmet is provided around the rim of the helmet.

Conveniently the helmet is of a partial double skin construction with the air inlet means including a plurality of apertures disposed around the periphery of the inner skin.

Preferably the spacing means is provided by projections fastened to the interior surface of the helmet, said projections being of a predetermined size so as to define an acceptable gap between the scalp and the helmet to permit air to flow there through. Alternatively said projections are adjustable relative to interior surfaces of helmet to permit the gap between the helmet and the scalp to be varied.

Conveniently the top of the helmet includes force coupling means by which a force can be applied to the helmet to maintain the helmet in constant contact with the scalp during treatment of the patient. Conveniently the coupling means is a projection disposed near the crown of the helmet.

The helmet is preferably provided together with cooling apparatus comprising cooling means for cooling a volume of air, fan means for supplying said cooled volume of air to the helmet, chamber means for recirculating the warmed air from such scalp through said cooling means, means coupled to the chamber means for reducing the moisture content entrained in said air flow, said fan means for recirculating cooled air through said helmet.

Preferably said cooling means comprises an evaporator, a compressor and an expansion valve in a refrigeration circuit.

Preferably the evaporator is disposed in said chamber means, said chamber means having an input plenum for receiving air warmed by the scalp, and which gradually increases in size from its inlet to the evaporator and an output plenum which decreases in size from the evaporator to a cool air outlet.

Preferably said input plenum includes a plurality of vanes coupled to the evaporator to distribute air flow over said evaporator surface, and said evaporator is coupled to a condensate drain.

Preferably also said fan means is a centrifugal fan disposed at said output plenum chamber cool air outlet or alternatively at said input plenum warm air input for supplying cooled air with a reduced moisture content to said helmet inlet means.

Preferably also said scalp cooling apparatus includes means for maintaining the helmet in gentle contact with the scalp. Conveniently said contact means are provided by a counterbalance arm having one end coupled to the helmet and the other end coupled to a counterweight. Alternatively said means of maintaining the helmet in contact with the scalp may be provided by coil springs or constant tension springs.

Preferably said chamber means includes a plenum assembly having an inlet plenum with an assembly inlet for receiving air to be cooled and dried, an outlet plenum for delivering cooled dried gas to an assembly outlet, and plate means separating the inlet and outlet plenum, said evaporation means being disposed between said inlet and outlet plenum.

Preferably said plenum assembly and said separate plate are generally horizontally disposed. Alternatively said plenum assembly and said separate plate is vertically disposed and said evaporator is disposed at the lowermost between said inlet and outlet plenums.

These and other aspects of the invention will become apparent from the following description when taken in combination with the accompanying drawings which :-
Fig. 1 is a diagrammatic view of a helmet in accordance with an embodiment of one aspect of the invention shown mounted in the head of a subject;
Fig. 2 is a diagrammatic sectional view of a plenum chamber for receiving air warmed by the scalp for reducing moisture content and for recirculating cooled air back to said scalp in accordance with an embodiment of another aspect of the present invention;
Fig. 3 is a top plan view of a receiving chamber shown in Fig. 2; and
Fig. 4 is a photograph of an embodiment of the invention shown in use on a patient.

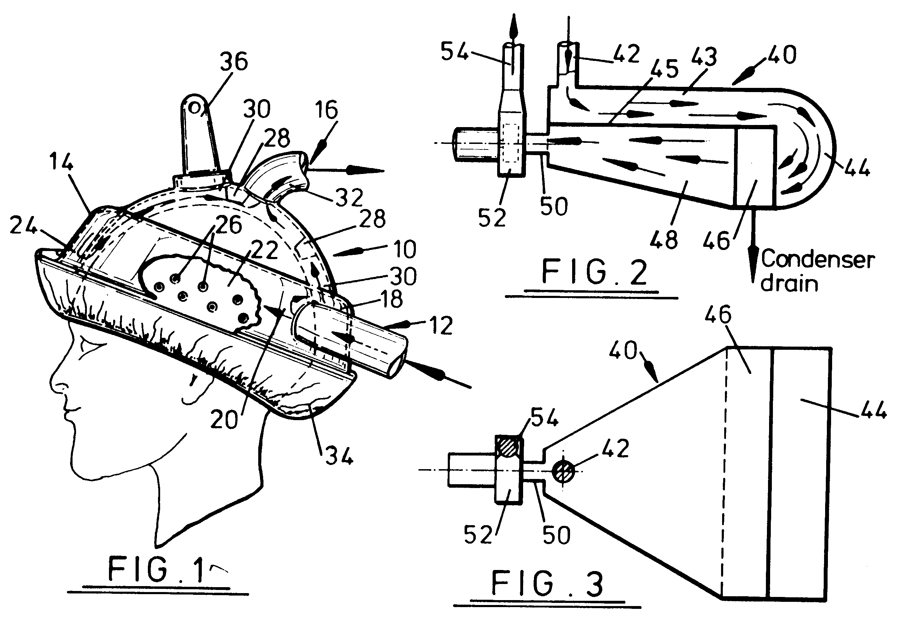

Reference is first made to Fig. 1 of the drawings which depicts a helmet generally indicated by the reference numeral 10 which is shown fitted on the head of a patent and which has an inlet 12 for receiving chilled air for cooling the scalp disposed at the rim 14 of the helmet and an air outlet 16 for returning air warmed by the scalp to a refrigeration unit for further cooling and reduction of moisture content as will be later explained in detail.

The helmet 10 is vacuum formed from plastic and is of a partial double skin construction in the vicinity of the rim 14. A flexible air inlet pipe or conduit 18 is coupled to the outer helmet skin 20 and air is supplied into a cavity 24 disposed between the inner and outer skins 22 and 20. Disposed around the periphery of the inner skin 22 are two rows of 6mm diameter apertures 26 which are dimensioned and chamfered so as to minimise frictional losses in the air stream. Thus air supplied at the inlet 12 through conduit 18 is fed around the periphery of the rim 14 between the inner and outer skins 20, 22 and through apertures 26 and onto the scalp in the vicinity of the hair line.

The interior helmet skin 22 is spaced from the scalp by a plurality of rubber projections 28 to define a gap 30 between the helmet and the scalp to permit cool air fed through the apertures 26 to circulate over the scalp.

Air circulating over the scalp is extracted at the crown of the helmet by flexible outlet conduit 32 which passes through the single helmet skin at the crown.

The rim of the helmet 14 has an elasticated fabric skirt 34 depending therefrom for sealing the helmet around the head of the subject. The fabric skirt restricts the entrainment of room air into the system and improves cooling air flow at the hair line margin. The fabric skirt also minimises any air loss from the closed circuit.

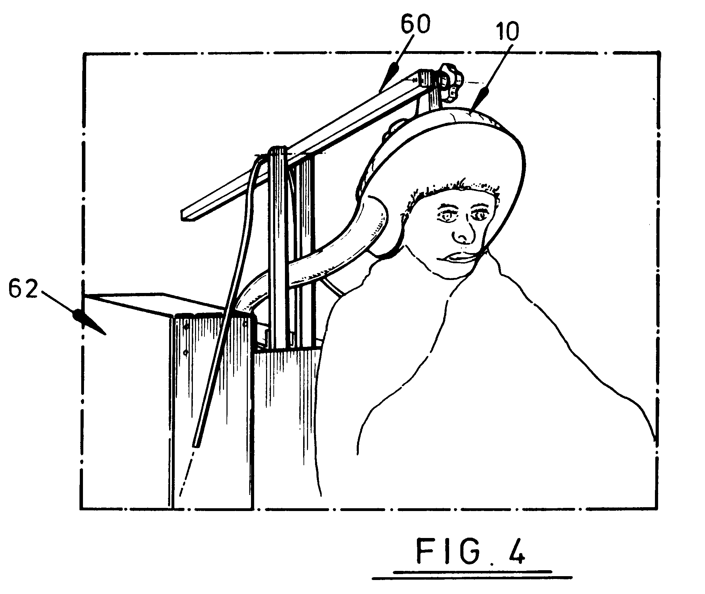

Near the crown of the helmet is coupled an upstanding projection 36 to which a counterbalance arm can be connected (as best seen in Fig. 4) for holding the helmet 10 in gentle contact with the scalp during treatment as will be later described in detail.

Reference is now made to Figs. 2 and 3 of the drawings which depicts a plenum assembly, generally indicated by a reference numeral 40, for receiving air warmed by the scalp and for reducing moisture content of the air and for recirculating cooled drier air to the helmet 10. The plenum assembly 40 has an inlet conduit 42 which is coupled to conduit 32 for receiving air warmed by the scalp. The inlet 42 feeds into an input plenum 43 which diverges from inlet 42 for guiding the warmed air over a plurality of air vanes 44 and then onto an evaporator indicated by reference numeral 46 which is a coil element from a 550 watt Searle TF2-0.3 cooler unit. The evaporator is coupled to a condensate drain from which condensed moisture can be removed.

The evaporator is also coupled to an output plenum 48 which converges towards an output plenum output 50. The output plenum is in thermal contact with the input plenum via separating plate 45. At the outlet 50 is coupled a single inlet centrifugal fan 52 of the free air type VBM4, Air Control Installation Limited and which has a free air supply of 76 cubic feet per minute. A fan outlet 54 is coupled to a conduit which supplies cooled drier air to the inlet conduit 18 coupled to the helmet 10.

It will be appreciated that both the input plenum 43 and the output plenum 48 are tapered or wedge-shaped so that initially the warm air flows through air inlet 42 and then diverges and flows over the separating plate 45 towards the full evaporator width 46. The plate 45 removes some moisture as does the evaporator 46 and chilled air from the evaporator then converges towards the plenum outlet 50. The tapered plenum design of both the input and the output plenums 43, 48 provides an impedance match between the delivery tubing 42, 54 and the evaporator orifice for both warm incoming air so that kinetic energy losses are minimised. Thus a sufficient volume of air can be recirculated through the closed system by the fan 54.

The plenum chamber 40 is part of the refrigeration circuit which includes a fridge compressor and an automatic expansion valve and, which are standard commercial components for room air chillers and the construction is in accordance with established techniques.

Reference is now made to the photograph shown in Fig. 4 of the drawings which depicts cooling apparatus in use or a patient. It will be seen that the helmet 10 is maintained in position by the counterbalanced arm 60 while the patient is seated in the chair. Flexible conduits connects the helmet 10 to the refrigeration unit, generally indicated by reference numeral 62, which includes the standard refrigeration components such as the compressor, evaporator, expansion valve centrifugal fan and condensate drain and some electrical switches.

In operation, the helmet 10 is disposed on a patient and the system is switched on so that the scalp is pre-cooled for around 10 minutes. Then drug infusion is performed for about 5 minutes and the patient then undergoes a period of treatment typically half an hour, because in this period the liver reduces the activity of the infused drug. The amount of pre-cooling, drug infusion, and post-cooling will vary from patient to patient and will depend on specific drugs however these figures are exemplary of a typical treatment regime.

When the system is switched on the air helmet directs cool air onto the scalp at the hair line through the apertures 26. The air then flows in the direction of the arrows shown in Fig. 1 over the scalp and out through outlet 16 back to the air recirculating plenum assembly 40. Because air is extracted at the crown of the helmet the convergence and increasing air velocity compensates for increased air temperature at this point due to heat being extracted from the scalp and thus scalp is optimised by using the wind-chill effect.

It will be appreciated that no control unit is required because, under normal ambient temperature conditions, the rate of heat flow from the air circulation circuit is limited by evaporator size and by the refrigerant used which can be any suitable refrigerant such as ammonia, ethyl chloride or Freon (trademark). With the system disclosed the air delivery between 11 - 27 cubic feet per minute at 0^{o}C and operational back pressures is obtained, and it has been found that the air delivery temperature at the helmet orifice is between -7^{o}C - -3^{o}C. The exhaust temperature at the helmet crown has been measured at about +3^{o}C. With the apparatus herein before described the cooling capacity was assessed by the use of a dummy scalp with uniform power dissipation in the form of a buried heated wire network and has been found to be 30 watts at 18^{o}C scalp temperature and 18 watts at 14^{o}C scalp temperature. It will also be appreciated under normal conditions of use the apparatus requires no defrosting cycle.

Various modifications may be made to the helmet and scalp cooling apparatus hereinbefore described without departing from the scope of the invention. For example the elasticated skirt may be omitted and the helmet may be maintained in position by means of coil springs or tensator springs instead of using counterbalance weights. The gap spacers inside the helmet can be rubber or plastic or any other suitable material which can gently contact the scalp which will permit a suitable flow of chilled air over the scalp during use. It will also be appreciated that although a compression refrigerator is described herein an absorption refrigerator without the compressor could be used instead in accordance with established technology. In addition the size and shape of the apertures 26 in the inner helmet skin may be varied as required to optimize air supply in cooling and the air outlet can be taken from a single or multiple point consistent with obtaining fixed scalp cooling. It will also be understood that the system could be used to supply air within a predetermined temperature range to other body sites for cooling such as the hands or feet. The plenum assembly could be disposed vertically so that the separating plate is vertical and the evaporator is located at the lowermost position between the plenum to facilitate condensation and moisture removal. An equivalent gas to air or air mixture could also be used with this apparatus.

Advantages of the system are that the helmet design is suitable for use with the majority of adult head sizes and does not require any adjustment except for the positioning of an elasticated fabric skirt at the periphery for restricting the entrainment of room air into the system and for improving cooling air flow at the hair line margin. The system does not require the use of control units or even a thermostat and under normal conditions of use the unit requires no defrosting cycle. In addition the system is capable of continuous running and requires minimal maintenance. The problem of freezing of entrained moisture on the evaporator is obviated, the circulating fan does not result in significant heat gain and the volume of air delivery is sufficient to cause satisfactory cooling of the scalp.

## Claims

1. A helmet (10) for use with a recirculating air scalp cooling system, said helmet (10) comprising air inlet means (12) and air outlet means (16), said air inlet means (12) being provided by a plurality of apertures (26) disposed around the periphery of the rim of the helmet and said air outlet means (16) being disposed at the crown of the helmet, characterised in that spacing means (28) are located inside the helmet for setting the distance between the helmet and the scalp and means (34) for minimising air loss from and to prevent entrainment of room air into the helmet is provided around the rim of the helmet.

2. A helmet as claimed in claim 1 wherein the helmet (10) is of a partial double skin construction (20, 22) with the air inlet means (12) including a plurality of apertures (26) disposed around the periphery of the inner skin (22).

3. A helmet as claimed in any preceding claim wherein the spacing means is provided by projections (28) fastened to the interior surface of the helmet, said projections (28) being of a predetermined size so as to define an acceptable gap between the scalp and the helmet to permit air to flow therethrough.

4. A helmet as claimed in claims 1 to 2 wherein said projections are adjustable relative to interior surfaces of helmet to permit the gap between the helmet and the scalp to be varied.

5. A helmet as claimed in any preceding claim wherein the top of the helmet includes force coupling means (36) by which a force can be applied to the helmet (10) to maintain the helmet in constant contact with the scalp during treatment of the patient.

6. A helmet as claimed in claim 5 wherein the coupling means is a projection (36) disposed near the crown of the helmet.

7. A helmet as claimed in any preceding claim in combination with cooling apparatus comprising cooling means (46) for cooling a volume of air, fan means (52) for supplying said cooled volume of air to the helmet (10), chamber means (40) for recirculating the warmed air from the scalp through said cooling means (46), means (45) coupled to the chamber means for reducing the moisture content entrained in said air flow, said fan means (52) for recirculating cooled air through said helmet (10).

8. A combination as claimed in claim 7 wherein said cooling means comprises an evaporator (46), a compressor and an expansion valve in a refrigeration circuit.

9. A combination as claimed in claim 8 wherein the evaporator (46) is disposed in said chamber means (40), said chamber means having an input plenum (43) for receiving air warmed by the scalp, and which gradually increases in size from its inlet (42) to the evaporator (46) and an output plenum (48) which decreases in size from the evaporator (46) to a cool air outlet (50).

10. A combination as claimed in claim 9 wherein said input plenum (43) includes a plurality of vanes (44) coupled to the evaporator (46) to distribute air flow over said evaporator surface, and said evaporator (46) is coupled to a condensate drain.

11. A combination as claimed in one of claims 9 or 10 wherein said fan means is a centrifugal fan (52) disposed at said output plenum chamber cool air outlet (50) or alternatively at said input plenum warm air input (42).

12. A combination as claimed in any one of claims 7 to 11 wherein said cooling apparatus includes means (60) for maintaining the helmet (10) in gentle contact with scalp.

13. A combination as claimed in claim 12 wherein said contact means are provided by a counterbalance arm (60) having one end coupled to the helmet (10) and the other end coupled to counterweight.

14. A combination as claimed in claim 12 wherein said means of maintaining the helmet in contact with the scalp may be provided by coil springs or constant tension springs.

15. A combination as claimed in any one of claims 8 to 14 wherein the chamber means includes a inlet plenum (43) having an assembly inlet (42) for receiving a supply of air to be cooled and dried, an output plenum (48) for delivering cooled dried air to an assembly outlet (50), a plate (45) separating the inlet and outlet plenum (43, 48) and evaporation means (46) disposed between said inlet and outlet plenums (43, 48).

16. A combination as claimed in claim 15 wherein said plenum assembly (40) and said plate (45) are generally horizontally disposed.

17. A plenum assembly as claimed in claim 15 wherein said plenum assembly and said plate are vertically disposed and said evaporator is disposed at the lowermost point between said inlet and outlet plenums.

## Patentansprüche

1. Helm (10) zur Verwendung mit einem Umluft-Kühlsystem für die Kopfhaut, wobei der Heim (10) Lufteinlaß-Mittel (12) und Luftauslaß-Mittel (16) aufweist, wobei die Lufteinlaß-Mittel (12) mit einer Mehrzahl von Öffnungen (26), die rund um den Umfang der Helmkante herum angeordnet sind, ausgestattet sind und die Luftauslaß-Mittel (16) an der Spitze des Helms angeordnet sind, dadurch gekennzeichnet, daß an der Innenseite des Helms Abstands-Mittel (28) angebracht sind, die den Abstand zwischen Helm und Kopfhaut bestimmen und daß rund um den Umfang der Helmkarte Mittel (34) vorgesehen sind, die Luftverluste aus und das Ansaugen von Raumluft in den Helm hinein minimieren.

2. Helm nach Anspruch 1, wobei der Helm (10) von teilweise doppelschaligem Aufbau (20, 22) ist, wobei die Lufteinlaß-Mittel (12) eine Mehrzahl von Öffnungen (26) umfassen, die rund um den Umfang der inneren Schale (22) angeordnet sind.

3. Helm nach einem der vorangehenden Ansprüche, wobei die Abstands-Mittel als Vorsprünge (28) vorgesehen sind, die an der inneren Oberfläche des Helms befestigt sind, und wobei die Vorsprünge (28) von vorbestimmter Größe sind, um so einen ausreichenden Abstand zwischen Kopfhaut und Helm, der eine Luftströmung dazwischen ermöglicht, zu definieren.

4. Helm nach Anspruch 1 oder 2, wobei die Vorsprünge bezogen auf die innere Oberfläche des Helms einstellbar sind, damit es möglich ist, den Abstand zwischen Helm und Kopfhaut zu variieren.

5. Heim nach einem der vorangehenden Ansprüche, wobei die Oberseite des Helms Kraftübertragungsmittel (36) aufweist, durch die Kraft auf den Helm (10) ausgeübt werden kann, um während der Behandlung des Patienten den Helm in ständiger Anlage an der Kopfhaut zu halten.

6. Helm nach Anspruch 5, wobei die Übertragungsmittel ein Vorsprung (36) sind, der nahe der Helmspitze angeordnet ist.

7. Helm nach einem der vorangehenden Ansprüche in Kombination mit einer Kühlvorrichtung, die Kühlmittel (46) zum Kühlen eines Luftvolumens, Ventilatormittel (52) zum Zuführen des gekühlten Luftvolumens zum Helm (10), Kammermittel (40) zum Rezirkulieren der erwärmten Luft von der Kopfhaut durch die Kühlmittel (46), und Mittel (45) zum Reduzieren der in der Luftströmung mitgeführten Feuchtigkeit aufweist, die mit den Kammermitteln (40) verbunden sind, wobei die Ventilatormittel (52) die gekühlte Luft durch den Helm (10) rezirkulieren.

8. Kombination nach Anspruch 7, wobei die Kühlmittel einen Verdampfer (46), einen Kompressor und ein Drosselventil in einem Kühlkreislauf umfassen.

9. Kombination nach Anspruch 8, bei der der Verdampfer (46) in den Kammermitteln (40) angeordnet ist, wobei die Kammermittel ein Ansaug-Luftverteilergehäuse (43) zum Aufnehmen von durch die Kopfhaut erwärmter Luft, das vom Einlaß (42) bis zum Verdampfer (46) allmählich größer wird und ein Auslaß-Luftverteilergehäuse (48) aufweisen, das vom Verdampfer (46) zum Kühlluft-Auslaß (50) kleiner wird.

10. Kombination nach Anspruch 9, wobei das Ansaug-Luftverteilergehäuse (43) eine Mehrzahl von Luftleitblechen (44) aufweist, die mit dem Verdampfer (46) verbunden sind, um die Luftströmung über die Oberfläche des Verdampfers zu verteilen und wobei der Verdampfer (46) mit einem Kondensatablauf verbunden ist.

11. Kombination nach einem der Ansprüche 9 oder 10, wobei die Ventilatormittel ein ein Radialgebläse (52) sind, das am Kühlluft-Auslaß (50) der Kammer des Auslaß-Luftverteilergehäuses oder alternativ am Warmluft-Einlaß (42) des Ansaug-Luftverteilergehäuses angeordnet ist.

12. Kombination nach einem der Ansprüche 7 bis 11, wobei die Kühlvorrichtung Mittel (60) aufweist, durch die der Helm (10) in sanfter Anlage an die Kopfhaut gehalten wird.

13. Kombination nach Anspruch 12, wobei die Kontaktmittel als Gegengewichts-Arm (60) vorgesehen sind, dessen eines Ende mit dem Helm (10) verbunden ist und dessen anderes Ende mit dem Gegengewicht verbunden ist.

14. Kombination nach Anspruch 12, wobei die Mittel zum Aufrechterhalten der Anlage des Helms an die Kopfhaut als Wickelfedern oder als Federn mit gleichbleibender Spannung vorgesehen sein können.

15. Kombination nach einem der Ansprüche 8 bis 14, wobei die Kammermittel ein Ansaug-Luftverteilergehäuse (43) mit angefügtem Einlaß (42) zum Aufnehmen der zugeführten, zu kühlenden und zu trocknenden Luft, ein Auslaß-Luftverteilergehäuse (48) zum Abgeben der gekühlten, getrockneten Luft in einen angefügten Auslaß (50), eine Platte (45) zum Trennen der Ansaug- und Auslaß-Luftverteilergehäuse (43, 48) und Verdampfer-Mittel (46) aufweist, die zwischen den Ansaug- und Auslaß-Luftverteilergehäusen (43,48) angeordnet sind.

16. Kombination nach Anspruch 15, wobei die Gehäuse-Anordnung (40) und die Platte (45) im wesentlichen horizontal angeordnet sind.

17. Gehäuse-Anordnung nach Anspruch 15, wobei die Gehäuse-Anordnung und die Platte senkrecht angeordnet sind und der Verdampfer an der untersten Stelle zwischen den Ansaug- und Auslaß-Luftverteilergehäusen angeordnet ist.

## Revendications

1. Casque (10) destiné à être utilisé avec un système de refroidissement du cuir chevelu à recirculation de l'air, ledit casque (10) comprenant des moyens (12) d'entrée de l'air et des moyens (16) de sortie de l'air, lesdits moyens (12) d'entrée de l'air étant pourvus d'une pluralité d'ouvertures (26) disposées autour de la périphérie du rebord du casque, et lesdits moyens (16) de sortie de l'air étant disposés au niveau du sommet du casque, caractérisé en ce que des moyens d'espacement (28) sont situés à l'intérieur du casque pour régler la distance entre le casque et le cuir chevelu, et des moyens (34) pour réduire la perte d'air à partir du casque et empêcher l'entraînement de l'air ambiant vers l'intérieur du casque sont disposés autour du rebord du casque.

2. Casque selon la revendication 1, dans lequel le casque (10) possède une structure partielle à double coque (20,22) comportant les moyens (12) d'entrée de l'air incluant une pluralité d'ouvertures (26) disposées sur la périphérie de la coque intérieure (22).

3. Casque selon l'une quelconque des revendications précédentes, dans lequel les moyens d'espacement sont équipés de parties saillantes (28) fixées sur la surface intérieure du casque, lesdites parties saillantes (28) possédant une taille prédéterminée de manière à définir un intervalle acceptable entre le cuir chevelu et le casque afin de permettre un passage d'air dans cet intervalle.

4. Casque selon les revendications 1 et 2, dans lequel lesdites parties saillantes sont réglables par rapport aux surfaces intérieures du casque afin de permettre une modification de l'intervalle entre le casque et le cuir chevelu.

5. Casque selon l'une quelconque des revendications précédentes, dans lequel le sommet du casque comprend des moyens d'accouplement forcé (36), grâce auxquels une force peut être appliquée au casque (10) pour maintenir ce dernier en permanence en contact avec le cuir chevelu pendant le traitement du patient.

6. Casque selon la revendication 5, dans lequel les moyens de couplage sont constitués par une partie saillante (36) disposée à proximité du sommet du casque.

7. Casque selon l'une quelconque des revendications précédentes en combinaison avec un dispositif de refroidissement comprenant des moyens de refroidissement (46) servant à refroidir un volume d'air, des moyens formant ventilateur (52) pour envoyer ledit volume d'air refroidi au casque (10), des moyens formant chambre (40) pour faire recirculer l'air chaud à partir du cuir chevelu à travers lesdits moyens de refroidissement (46), des moyens (45) couplés aux moyens formant chambre pour réduire la teneur en humidité entraînée par ledit écoulement d'air, lesdits moyens formant ventilateur (52) pour mettre en recirculation l'air refroidi, à l'intérieur dudit casque (10).

8. Combinaison selon la revendication 7, dans laquelle lesdits moyens de refroidissement comprennent un évaporateur (46), un compresseur et une soupape de détente dans un circuit de réfrigération.

9. Combinaison selon la revendication 8, dans laquelle l'évaporateur (46) est disposé dans lesdits moyens formant chambre (40), lesdits moyens formant chambre comportant un collecteur d'entrée (43) servant à recevoir l'air chauffé par le cuir chevelu et dont la taille augmente graduellement depuis son entrée (42) en direction de l'évaporateur (46), et un collecteur de sortie (48), dont la taille diminue depuis l'évaporateur (46) en direction d'une sortie (50) de l'air froid.

10. Combinaison selon la revendication 9, dans laquelle ledit collecteur d'entrée (43) comprend une pluralité d'ailettes (44) couplées à l'évaporateur (46) pour répartir le courant d'air sur ladite surface de l'évaporateur, ledit évaporateur (46) est couplé à un système d'évacuation du condensat.

11. Combinaison selon l'une quelconque des revendications 9 ou 10, dans laquelle lesdits moyens formant ventilateur sont constitués par un ventilateur centrifuge (52) disposé dans ladite sortie (50) pour l'air froid de la chambre du collecteur de sortie ou, sinon, dans ladite entrée (42) pour l'air chaud du collecteur d'entrée.

12. Combinaison selon l'une quelconque des revendications 7 à 11, dans laquelle ledit dispositif de refroidissement comprend des moyens (60) pour maintenir le casque (10) selon un contact doux avec le cuir chevelu.

13. Combinaison selon la revendication 12, dans laquelle lesdits moyens de contact sont constitués par un bras d'équilibrage (60), dont une extrémité est couplée au casque (10), tandis que l'autre extrémité est accouplée au contrepoids.

14. Combinaison selon la revendication 12, dans laquelle lesdits moyens de maintien du casque en contact avec le cuir chevelu peuvent être formés par des ressorts hélicoïdaux ou des ressorts à tension constante.

15. Combinaison selon l'une quelconque des revendications 8 à 14, dans laquelle les moyens formant chambre contiennent un collecteur d'entrée (43) possédant une entrée (42) de l'ensemble de collecteur servant à recevoir une alimentation d'air devant être refroidi et séché, un collecteur de sortie (48) servant à délivrer de l'air sec refroidi à une sortie (50) de l'ensemble de collecteur, une plaque (45) séparant les collecteurs d'entrée et de sortie (43,48), et des moyens d'évaporation (46) disposés entre lesdits collecteurs d'entrée et de sortie (43,48).

16. Combinaison selon la revendication 15, dans laquelle ledit ensemble de collecteur (40) et ladite plaque (45) sont disposés d'une manière générale horizontalement.

17. Ensemble de collecteur selon la revendication 15, dans lequel ledit ensemble de collecteur et ladite coque sont disposés verticalement et ledit évaporateur est disposé au point le plus bas entre lesdits collecteurs d'entrée et de sortie.
